(19) Europäisches Patentamt
European Patent Office
Office européen des brevets

(11) Veröffentlichungsnummer: **0 210 120**
**B1**

(12) # EUROPÄISCHE PATENTSCHRIFT

(45) Veröffentlichungstag der Patentschrift: **10.05.89**

(51) Int. Cl.⁴: **H01F 7/02**, G01N 24/06, A61B 5/05

(21) Anmeldenummer: **86730083.2**

(22) Anmeldetag: **16.05.86**

(54) **Magnet eines Kernspintomographen.**

(30) Priorität: **23.05.85 DE 3518852**

(43) Veröffentlichungstag der Anmeldung: **28.01.87 Patentblatt 87/5**

(45) Bekanntmachung des Hinweises auf die Patenterteilung: **10.05.89 Patentblatt 89/19**

(84) Benannte Vertragsstaaten: **DE FR**

(56) Entgegenhaltungen:
**EP-A- 0 170 318**
**WO-A-84/01226**
**DE-A- 2 605 666**
**DE-B- 1 283 976**
**FR-A- 985 815**
**FR-A- 1 047 700**
**FR-A- 1 125 874**
**FR-A- 2 549 281**
**FR-A- 2 562 785**
**NL-A- 298 777**
**US-A- 3 927 397**

**IBM TECHNICAL DISCLOSURE BULLETIN, Band 19, Nr. 5, Oktober 1976, Seiten 1856-1857, New York, US; R.E. JONES, Jr. et al.: "Directionally homogeneous magnets for vacuum-deposition apparatus"**

(73) Patentinhaber: **Siemens Aktiengesellschaft Berlin und München, Wittelsbacherplatz 2, D-8000 München 2(DE)**

(72) Erfinder: **Schwab, August, Dr. Techn., Am Laubwald 10, D-1000 Berlin 13(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 210 120 B1

## Beschreibung

Die Erfindung bezieht sich auf einen Magneten eines Kernspintomographen aus magnetisiertem Dauermagnetwerkstoff mit einer sich in Längsrichtung erstreckenden, den Innenraum zur Aufnahme eines Patienten bildenden Öffnung, der im Innenraum ein senkrecht zur Längsachse des Magneten stehendes, angenähert homogenes Magnetfeld erzeugt.

Ein derartiger aus der WO 8 401 226 bekannter Magnet .erzeugt ein homogenes Magnetfeld, dessen relative Inhomogenität $5.10^{-4}$ beträgt. Außerhalb des Magneten herrscht nur ein geringes Streufeld, von dem der Hauptteil im Bereich der Stirnseiten des Magneten verläuft. Der bekannte Magnet ist aus in Längsrichtung hintereinander liegenden angenähert kreisförmigen Ringen zusammengesetzt. Die Ringe haben unterschiedliche Innendurchmesser, die zur Mitte des Magneten hin zunehmen, wodurch die Homogenität des Magnetfeldes im Innenraum erhöht wird und eine geringere Baulänge als bei einem Magneten mit konstantem Innendurchmesser erzielt wird. Jeder Ring besteht aus mehreren gleichartigen trapezförmigen magnetisierten Segmenten, derart, daß der Querschnitt der Öffnung des Magneten und seiner äußeren Kontur ein gleichseitiges Vieleck sind, wodurch der Magnet angenähert die Form eines Hohlzylinders hat. Während der Montage sind zur Einstellung der Lage der Segmente Fugen zwischen ihnen notwendig. Die Segmente eines Ringes berühren einander also nicht. Wegen dieser Fugen erhält man jedoch im Innenraum des Magneten nicht die Stärke des Magnetfeldes, die man im Innenraum bei einem hohlzylindrischen fugenlosen Magneten aus Dauermagnetwerkstoff erhält, da in den notwendigen Fugen zwischen den Segmenten ein magnetischer Spannungsabfall auftritt. Auch ist das durch die Fugen bedingte unerwünschte Streufeld im Bereich der Mantelfläche größer.

Die Herstellung des bekannten Magneten ist aufwendig, insbesondere ist die Magnetisierung der Segmente und deren Montage zu einem Ring kompliziert.

Die Segmente bestehen aus einem Dauermagnetwerkstoff mit Vorzugsrichtung. Jedes Segment wird in Vorzugsrichtung magnetisiert, bevor die Segmente zu einem Ring zusammengefügt werden. Dabei ist die Vorzugsrichtung in jedem Segment unterschiedlich, sie ist abhängig vom Ort der späteren Lage des Mittelpunktes des Segmentes. Jedes Segment muß also gesondert magnetisiert werden.

Jedes bereits magnetisierte Segment wird zur Montage von einem Rahmen gehalten, der eine Feineinstellschraube aufweist, mit der die endgültige Lage des Segmentes eingestellt werden kann. Zur Montage werden alle Rahmen mit den Segmenten mittels spezieller Führungen in ihre Endposition gebracht. Dabei treten große magnetische Kräfte zwischen den Segmenten auf, die von den Führungen aufgefangen werden müssen. Auch beeinflussen sich die Magnetfelder der Segmente während der zur Montage notwendigen Bewegungen gegenseitig, so daß das Magnetfeld im Innenraum zur Aufnahme eines Patienten nicht ausreichend homogen sein kann. Zur Korrektur des Magnetfeldes im Innenraum werden die Lagen aller Segmente mittels der Feineinstellschrauben verändert, wobei für jedes Segment mehrere Messungen und Korrekturrechnungen durch eine Datenverarbeitungsanlage notwendig sind. Nachdem diese Korrekturen beendet sind, werden die Segmente in ihrer Lage fixiert und die Rahmen entfernt. Auf diese Art ist einer der hintereinanderliegenden Ringe fertiggestellt. Anschließend wird dieser Ring in einem Stützrahmen eingelassen. Diese Prozedur wird für jeden folgenden Ring wiederholt und durch weitere Messungen und Rechnungen wird die Lage der Ringe im Stützrahmen festgelegt.

Der Erfindung liegt die Aufgabe zugrunde, einen Magneten aus Dauermagnetwerkstoff mit homogenem Feld der eingangs beschriebenen Art zu schaffen, dessen Herstellung vereinfacht ist.

Zur Lösung der Aufgabe ist gemäß der Erfindung ein Magnet so ausgebildet, daß die Öffnung und die äußere Kontur des Magneten im wesentlichen rechteckförmig sind, wodurch jeweils zwei parallel und zwei senkrecht zum angenähert homogenen Magnetfeld im Innenraum verlaufende, den Innenraum begrenzende Schenkel (Vertikalschenkel, Horizontalschenkel) gebildet sind, die durch vier Verbindungsteile verbunden sind, und der Magnet ist nach seiner Montage als Ganzes magnetisiert derart, daß das Magnetfeld in den Vertikalschenkeln hauptsächlich parallel aber entgegengerichtet zum Magnetfeld im Innenraum verläuft und sich über die Horizontalschenkel und die Verbindungsteile schließt, wobei die magnetische Flußdichte von den inneren Flächen der Vertikalschenkel zu den äußeren Flächen der Vertikalschenkel hin zunimmt. Weil die Öffnung und die äußere Kontur im wesentlichen rechteckförmig sind, hat ein derartiger Magnet eine geometrisch einfache Form, die sich günstig aus handelsüblichen, quaderförmigen Blöcken zusammensetzen läßt. Wegen der fehlenden Fugen in Umfangsrichtung ist die Ausnutzung des Dauermagnetwerkstoffs hoch, d.h. im Innenraum herrscht ein starkes Magnetfeld, und das Streufeld im Außenraum ist gering. Da der Dauermagnetwerkstoff bei der Montage unmagnetisch ist, treten während der Montage keine magnetischen Kräfte zwischen Teilen des Magneten auf und somit sind keine aufwendigen Halterungen nötig. Da das Magnetfeld in den Vertikalschenkeln hauptsächlich parallel, aber entgegengerichtet zum homogenen Feld im Innenraum verläuft und sich über die Horizontalschenkel und die quaderförmigen Verbindungsstücke schließt, wobei die magnetische Flußdichte von den inneren Flächen der Vertikalschenkel zu den äußeren Flächen hin zunimmt, ist der Außenraum des Magneten angenähert feldfrei. Das bedeutet, daß der vom Dauermagnetwerkstoff hervorgerufene magnetische Fluß hauptsächlich den Innenraum des Magneten und kaum den Außenraum durchsetzt.

Es ist vorteilhaft, wenn an der Innenfläche jedes Horizontalschenkels eine weichmagnetische Platte angeordnet ist, die an ihrer dem Innenraum zugewandten Oberfläche weichmagnetische, dünne, schmale Blechstreifen aufweist, die nicht die gesamte Oberfläche der weichmagnetischen Platten bedecken. Dann wird die Homogenität des Magnetfeldes im Innenraum unterstützt, denn die weichmagnetischen Platten erzwingen Flächen gleicher magnetischer Spannung. Durch dünne, schmale, an der dem Innenraum zugewendeten Oberfläche der weichmagnetischen Platten angeordnete, weichmagnetische Blechstreifen, die nicht die gesamte Oberfläche der weichmagnetischen Platten bedecken, können nach der Aufmagnetisierung kleine Inhomogenitäten des Magnetfeldes ausgeglichen werden. Die weichmagnetischen Platten können besonders einfach aus Blechen hergestellt sein, wobei die Bleche senkrecht zur Längsrichtung des Magneten verlaufen, wodurch eine Formgebung der Platten erleichtert ist. Es empfiehlt sich, daß wenigstens eine der weichmagnetischen Platten an der dem Innenraum zugewandten Oberfläche eine quer zur Längsrichtung verlaufende konkave Wölbung aufweist. Die Wölbung erhöht die Homogenität des Magnetfeldes im Innenraum, wodurch die Baulänge des Magneten verkürzt werden kann. Der Bedarf an Magnetmaterial wird verringert, wenn der Magnet aus einem Magnetwerkstoff mit Vorzugsrichtung besteht.

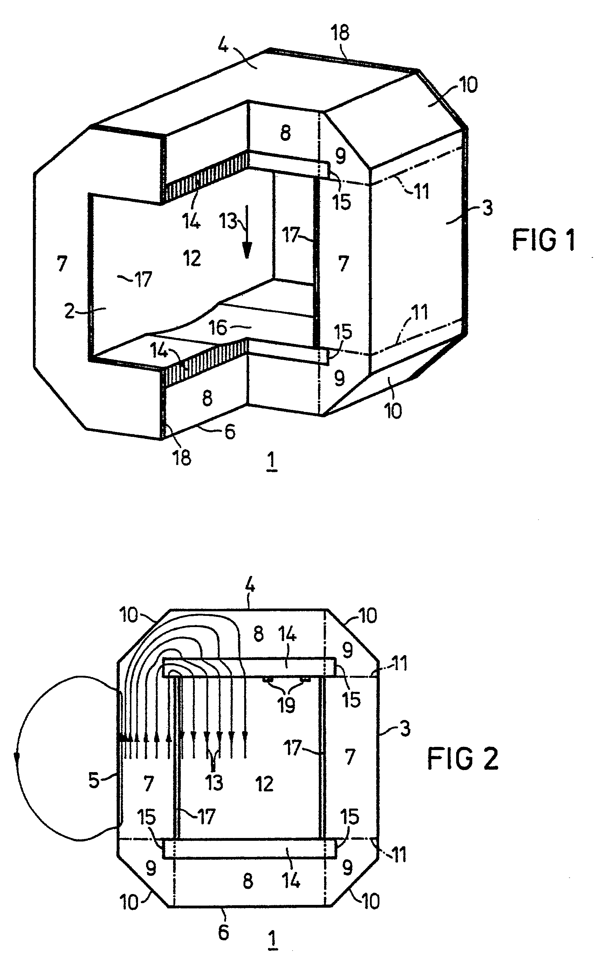

Im folgenden sei die Erfindung noch anhand des in den Figuren 1 und 2 der Zeichnung dargestellten Ausführungsbeispiels näher erläutert.

Figur 1 zeigt eine perspektivische Ansicht auf einen zum Teil geschnittenen, gemäß der Erfindung ausgebildeten Magneten. In Figur 2 ist der Feldverlauf im Magneten in einer quer zur Längsrichtung verlaufenden Ebene dargestellt.

Da die Querachsen des Magneten Symmetrielinien des Feldverlaufes sind, ist der Feldverlauf zur Vereinfachung der Darstellung nur in einem Viertel des Magneten dargestellt.

Der Magnet 1 eines Kernspintomographen ist im wesentlichen quaderförmig und hat eine zentrale, sich in Längsrichtung des Magneten 1 erstreckende, durchgehende, rechteckförmige Öffnung 2, die parallel zu den äußeren Flächen 3, 4, 5, 6 des Magneten 1 verläuft. Dadurch setzt sich der Magnet 1 zusammen aus zwei quaderförmigen Vertikalschenkeln 7, zwei quaderförmigen Horizontalschenkeln 8 und den sie verbindenden vier Verbindungsteilen 9. Der Magnet 1 kann aus einem Keramik-Ferrit-Dauermagnetwerkstoff bestehen, der in handelsüblichen quaderförmigen Blöcken unmagnetisiert verarbeitet ist. Die Vertikalschenkel 7 und die Horizontalschenkel 8 sind einfach durch Verkleben der quaderförmigen Blöcke ohne Nachbearbeitung hergestellt. Die Verbindungsteile 9 sind außen abgeschrägt. Ihre schräg verlaufenden Flächen 10 verbinden die äußeren Flächen 3, 4, 5, 6. Durch die Schrägung ist die äußere Kontur des Magneten 1 an den optimalen Feldverlauf angepaßt, wodurch Dauermagnetwerkstoff eingespart wird.

Die Vertikalschenkel 7, die Horizontalschenkel 8 und die Verbindungsteile 9 sind an ihren gegenseitigen Berührungsflächen 11, durch strichpunktierte Linien dargestellt, verklebt, dadurch ist die Montage des Magneten 1 einfach. Somit umgibt der magnetisierte Dauermagnetwerkstoff fugenlos den durch die durchgehende Öffnung 2 gebildeten Innenraum 12, der zur Aufnahme einer nicht dargestellten erwachsenen Person geeignet ist. Im Innenraum 12 herrscht ein senkrecht zur Längsachse des Magneten 1 stehendes angenähert homogenes Magnetfeld 13.

Zur Homogenisierung des Magnetfeldes 13 sind senkrecht zum Magnetfeld 13 verlaufende, weichmagnetische Platten 14 an den Innenflächen der Horizontalschenkel 8 angebracht. Die beiden weichmagnetischen Platten 14 sind breiter als die Horizontalschenkel 8 und in Ausnehmungen 15 der Verbindungsteile 9, durch gepunktete Linien dargestellt, eingelassen. Man erhält die Ausnehmungen 15 in einfacher Weise durch Fortlassen von quaderförmigen Blöcken aus Dauermagnetwerkstoff an den den Flächen 10 gegenüberliegenden Ecken. Damit fallen alle von der Quaderform abweichenden Bereiche des Magneten 1 in die Verbindungsteile 9, so daß Bearbeitungen nur an diesen vorgenommen werden müssen. Dies ist für eine einfache Herstellung des Magneten 1 wichtig, denn der Dauermagnetwerkstoff ist mechanisch sehr hart, und Nachbearbeitungen sind daher sehr aufwendig.

Die beiden weichmagnetischen Platten 14 bestehen aus in Längsrichtung hintereinander liegenden Blechen. Die untere der beiden weichmagnetischen Platten 14 weist eine konkave Wölbung 16 auf, die zur Homogenisierung des Magnetfeldes 13 beiträgt, so daß die Baulänge gegenüber einem Magneten ohne Wölbung verringert ist. Die Wölbung 16 ergibt sich dadurch, daß hintereinanderfolgende Bleche eine zur Mitte des Magneten hin abnehmende Höhe aufweisen. Die Blechung der weichmagnetischen Platten 14 trägt somit zur einfachen Herstellung des Magneten 1 bei, da bei massiv ausgeführten Platten eine Nachbearbeitung zur Erzielung der Wölbung notwendig wäre. Ferner ist durch die Anordnung der weichmagnetischen Platten 14 an den Horizontalschenkeln 8 die Herstellung des Magneten 1 vereinfacht, da die Wölbung 16 an ihnen einfacher herzustellen ist als an den mechanisch harten Horizontalschenkeln 8 aus Dauermagnetwerkstoff.

Die zwischen den beiden weichmagnetischen Platten 14 wirkenden magnetischen Kräfte werden durch nichtmagnetische Stützen 17 aufgefangen. Sie sind an den Innenflächen der Vertikalschenkel 7 angeordnet und an den Stirnseiten des Magneten 1 mit nichtmagnetischen Rahmen 18 verbunden.

Wie Fig. 2 zeigt, ist der aus einem Keramik-Ferrit-Dauermagnetwerkstoff bestehende Magnet 1 so magnetisiert, daß das Magnetfeld fast ausschließlich in den Vertikalschenkeln 7, den Verbindungsteilen 9, den Horizontalschenkeln 8, den weichmagnetischen Platten 14 und im Innenraum 12 des Magneten 1 verläuft. Der Außenraum des Magneten 1 ist angenähert feldfrei. Im Innenraum 7 herrscht ein homogenes Magnetfeld 13 von 0,2 T, während das Magnetfeld im Außenraum des Magneten 1 in 0,5 m Entfernung von den Flächen 3, 5 nur noch 0,01 T beträgt. In den Vertikalschenkeln 7 verläuft das Magnetfeld hauptsächlich parallel, aber entgegengerichtet zum Magnetfeld 13 im Innenraum 12. Dabei nimmt die magnetische Flußdichte von den inneren Flächen der Vertikalschenkel 7 zu den äußeren Flächen 3, 5 hin zu, so daß die Flußdichte dort fast den Wert der Remanenzinduktion des Keramik-Ferrit-Dauermagnetwerkstoffs erreicht. Auch an den anderen äußeren Flächen 4, 6 und den Flächen 10, die alle Grenzflächen zwischen unterschiedlichen Werkstoffen darstellen, verläuft das Feld angenähert parallel zu diesen Grenzflächen. Die Flußdichte nimmt dort den Wert der Remanenzinduktion des Keramik-Ferrit-Dauermagnetwerkstoffs an. Das bedeutet, daß dort die magnetische Feldstärke angenähert Null ist, so daß der Außenraum des Magneten 1 aufgrund der Bedingungen an Grenzflächen zwischen unterschiedlichen magnetischen Werkstoffen angenähert feldfrei ist.

Ein Teil des magnetischen Flusses in den Vertikalschenkeln 7 verläuft über den Teil der weichmagnetischen Platten 14, der in den Ausnehmungen 15 der Verbindungsteile 9 eingelassen ist. Dadurch sind die Verbindungsteile 11 und die Horizontalschenkel 8 magnetisch entlastet, so daß die Horizontalschenkel 8 schmaler als die Vertikalschenkel 7 sind, um eine ähnliche hohe Ausnutzung des Keramik-Ferrit-Dauermagnetwerkstoffs wie in den Vertikalschenkeln 7 zu erzielen. Die weichmagnetischen Platten 14 tragen also auch zu einer Einsparung von Keramik-Ferrit-Dauermagnetwerkstoff bei. Ein Ersatz des Keramik-Fertig-Dauermagnetwerkstoffs in den Horizontalschenkeln 8 und den Verbindungsteilen 9 durch weichmagnetische Werkstoffe hätte jedoch den Nachteil, daß bei gleichen äußeren Abmessungen im Innenraum 12 ein schwächeres Magnetfeld herrschen würde, da die Horizontalschenkel 8 nicht zum Aufbau des Magnetfeldes beitragen würden, und das Streufeld im Außenraum des Magneten 1 wäre vergrößert.

Der Magnet 1 ist nach seiner Montage als Ganzes durch eine nicht dargestellte einfache quaderförmige Spule mittels eines Stromimpulses aufmagnetisiert. Dabei wird im Innenraum 12 ein homogenes Magnetfeld mit einer Flußdichte von 2,7 T erreicht, und im Magneten 1 wird die Remanenzinduktion des Keramik-Ferrit-Dauermagnetwerkstoffs überschritten.

Da örtliche Schwankungen der Eigenschaften des Keramik-Ferrit-Dauermagnetwerkstoffs innerhalb der Teile des Magneten 1 nicht zu vermeiden sind, welche die Inhomogenität über den geforderten Wert ansteigen lassen, sind zur Korrektur des Magnetfeldes 13 im Innenraum 12 dünne, schmale Blechstreifen 19 an den Innenflächen der weichmagnetischen Platten 14 angebracht. Sie sind in Fig. 2 nur an der oberen Platte 14 beispielhaft dargestellt, da ihre Abmessungen, ihre Ausrichtung und ihre örtliche Lage auf der Oberfläche der Platten 14 von zufälligen Schwankungen der Magnetwerkstoffeigenschaften abhängt. Ihre Lage wird nach der Aufmagnetisierung des Magneten 1 aufgrund einer Messung des Magnetfeldes 13 durch Rechnung ermittelt. Die genaue Lage kann aber auch durch einfache Versuche anstelle der Rechnung festgestellt werden.

Die Ausführung des Magneten gemäß der Erfindung ist nicht auf Keramik-Ferrit-Dauermagnetwerkstoff beschränkt. Es können auch höherremanente Werkstoffe verwendet werden, z.B. auf Sm-Co-Basis, um höhere Flußdichten im Innenraum 12 zu erreichen. Dabei können auch Dauermagnetwerkstoffe mit Vorzugsrichtung verwendet werden.

## Patentansprüche

1. Magnet eines Kernspintomographen aus magnetisiertem Dauermagnetwerkstoff mit einer sich in Längsrichtung erstreckenden, den Innenraum (12) zur Aufnahme eines Patienten bildenden Öffnung, der im Innenraum (12) ein senkrecht zur Längsachse des Magneten stehendes, angenähert homogenes Magnetfeld erzeugt, dadurch gekennzeichnet, daß die Öffnung (2) und die äußere Kontur des Magneten (1) im wesentlichen rechteckförmig sind, wodurch jeweils zwei parallel und zwei senkrecht zum angenähert homogenen Magnetfeld (13) im Innenraum (12) verlaufende, den Innenraum (12) begrenzende Schenkel (Vertikalschenkel 7, Horizontalschenkel 8) gebildet sind, die durch vier Verbindungsteile (9) verbunden sind, und daß der Magnet (1) als Ganzes nach seiner Montage derart magnetisiert ist, daß das Magnetfeld in den Vertikalschenkeln (7) hauptsächlich parallel, aber entgegengerichtet zum Magnetfeld (13) im Innenraum (12) verläuft und sich über die Horizontalschenkel (8) und die Verbindungsteile (9) schließt, wobei die magnetische Flußdichte von den inneren Flächen der Vertikalschenkel (7) zu den äußeren Flächen (3, 5) der Vertikalschenkel (7) hin zunimmt.

2. Magnet nach Anspruch 1, dadurch gekennzeichnet, daß an der Innenfläche jedes Horizontalschenkels (8) eine weichmagnetische Platte (14) angeordnet ist, die an ihrer dem Innenraum (12) zugewandten Oberfläche weichmagnetische, dünne, schmale Blechstreifen (19) aufweist, die nicht die gesamte Oberfläche der weichmagnetischen Platten (14) bedecken.

3. Magnet nach Anspruch 2, dadurch gekennzeichnet, daß die weichmagnetischen Platten (14) geblecht sind, wobei die Bleche senkrecht zur Längsrichtung des Magneten (1) verlaufen.

4. Magnet nach Anspruch 2 oder 3, dadurch gekennzeichnet, daß wenigstens eine der weichmagnetischen Platten (14) an der dem Innenraum (12) zugewandten Oberfläche eine quer zur Längsrichtung verlaufende konkave Wölbung (16) aufweist.

5. Magnet nach Anspruch 1, dadurch gekennzeichnet, daß die Verbindungsteile (9) abgeschrägt sind.

6. Magnet nach Anspruch 1, dadurch gekennzeichnet, daß der Magnet (1) aus einem Magnetwerkstoff mit Vorzugsrichtung besteht.

## Claims

1. Magnet for a nuclear magnetic resonance tomograph of magnetised permanent magnet material having an aperture extending in the longitudinal direction and forming the inner chamber (12) for receiving a patient, which produces in the inner chamber (12) an approximately homogeneous magnetic field perpendicular to the longitudinal axis of the magnet, characterised in that the aperture (2) and the outer contour of the magnet (1) are substantially rectangular in shape, so that two members are formed extending parallel to the approximately homogeneous magnetic field (13) in the inner chamber (12), and two extending perpendicular toit (vertical members 7, horizontal members 8), defining the inner chamber (12), these members being joined by four connecting parts (9), and in that the magnet (1) as a whole is magnetised after its assembly so that the magnetic field in the vertical arms (7) extends for the most part parallel, but in the opposite direction to the magnetic field (13) in the inner chamber (12) and closes via the horizontal members (8) and the connecting parts (9), the magnetic flux density increasing from the inner surfaces of the vertical members (7) to the outer surfaces (3, 5) of the vertical members (7).

2. Magnet according to claim 1, characterised in that on the inner surface of each horizontal member (8) there is arranged a soft magnetic plate (14) which has on its surface facing the inner chamber (12) soft magnetic, thin narrow sheet metal strips (19) which do not cover the entire surface of the soft magnetic plates (14).

3. Magnet according to claim 2, characterised in that the soft magnetic plates (14) are laminated and the laminations extend perpendicularly to the longitudinal direction of the magnet (1).

4. Magnet according to claim 2 or 3, characterised in that at least one of the soft magnetic plates (14) has on the surface facing the inner chamber (12) a concavity (16) extending transversely to the longitudinal direction.

5. Magnet according to claim 1, characterised in that the connecting parts (9) are chamfered.

6. Magnet according to claim 1, characterised in that the magnet (1) comprises a magnetic material with a preferred direction.

## Revendications

1. Aimant d'un tomographe à résonance magnétique nucléaire fait avec un matériau aimanté, à aimantation permanente, avec une ouverture qui s'étend dans le sens longitudinal et qui forme la chambre intérieure (12) destinée à recevoir un patient et produisant dans ladite chambre intérieure (12), un champ magnétique sensiblement homogène et perpendiculaire à l'axe longitudinal, caractérisé par le fait que l'ouverture (2) et le contour extérieur de l'aimant ont sensiblement la forme d'un rectangle, ce qui conduit à la formation de respectivement deux branches parallèles et deux branches perpendiculaires au champ magnétique (13) sensiblement homogène dans la chambre intérieure (12), lesquelles branches (branches verticales 7, branches horizontales 8) qui délimitent ladite chambre intérieure (12) sont reliées par quatre éléments de liaison (9), et que l'aimant (1) est, après son montage et dans son ensemble, aimanté de telle façon que le champ magnétique s'étend dans les branches verticales (7) principalement parallèlement, mais en sens opposé au champ magnétique (13) qui règne dans la chambre intérieure (12), et se ferme par l'intermédiaire des branches horizontales (8) et les éléments de liaison (9), la densité du flux magnétique augmentant depuis les surfaces intérieures des branches verticales jusqu'aux surfaces extérieures (3, 5) des branches verticales (7).

2. Aimant selon la revendication 1, caractérisé par le fait que contre la surface intérieure de chaque branche horizontale (8) est placée une plaque (14) faite avec un matériau magnétique doux et qui comporte, sur sa surface tournée vers la chambre intérieure (12), des branches de tôle minces et étroites (19) faites avec un matériau magnétique doux et ne recouvrant pas la totalité de la surface des plaques (14) faites avec un matériau magnétique doux.

3. Aimant selon la revendication 2, caractérisé par le fait que les plaques (14) faites avec un matériau magnétique doux sont feuilletées, les tôles s'étendant perpendiculairement à la direction longitudinale de l'aimant (1).

4. Aimant selon la revendication 2 ou 3, caractérisé par le fait qu'au moins l'une des plaques (14) faites avec un matériau magnétique doux comporte, sur la surface tournée vers la chambre intérieure (12), une voûte concave (16) s'étendant transversalement par rapport à la direction longitudinale.

5. Aimant selon la revendication 1, caractérisé par le fait que les éléments de liaison (9) sont chanfreinés.

6. Aimant selon la revendication 1, caractérisé par le fait que l'aimant (1) est constitué avec un matériau magnétique à direction préférentielle.

4
18
10
8
9
15
11
3
14
13
17
12
7
2
16
11
15
14
9
8
10
6
18
1
FIG 1

4
10
8
14
10
9
11
15
19
3
17
7
5
7
13
12
15
17
15
11
9
8
14
9
10
10
6
1
FIG 2